# EUROPÄISCHE PATENTSCHRIFT

(11) **EP 2 906 914 B1**
(45) Veröffentlichungstag und Bekanntmachung des Hinweises auf die Patenterteilung: **29.04.2020**
(21) Anmeldenummer: 13762144.7
(22) Anmeldetag: 16.09.2013
(51) Int. Cl.: F02D 41/14, G01N 27/12, G01N 27/22, G01F 5/00, F02D 41/18, F02M 35/10, G01N 33/00

(54) **SENSORANORDNUNG ZUR BESTIMMUNG DES FEUCHTEGEHALTS EINES IN EINER HAUPTSTRÖMUNGSRICHTUNG STRÖMENDEN FLUIDEN MEDIUMS**
SENSOR DEVICE FOR DETERMINING THE MOISTURE CONTENT OF A FLUID MEDIUM FLOWING IN A MAIN FLOW DIRECTION
AGENCEMENT DE CAPTEUR POUR DÉTERMINER LE CONTENU EN HUMIDITÉ D'UN MILIEU DE FLUIDE EN ÉCOULEMENT DANS LA DIRECTION PRINCIPALE DU L'ÉCOULEMENT

(30) Priorität: 15.10.2012 DE 102012218758
(43) Veröffentlichungstag der Anmeldung: 19.08.2015
(73) Patentinhaber: Robert Bosch GmbH, 70442 Stuttgart (DE)
(72) Erfinder: BRIESE, Achim, 71277 Rutesheim (DE); HAGMANNS, Lukas, 76137 Karlsruhe (DE); WAGNER, Ulrich, 80689 Muenchen (DE); KAUFMANN, Andreas, 89567 Sontheim An Der Brenz (DE)
(86) Internationale Anmeldenummer: PCT/EP2013/069132
(87) Internationale Veröffentlichungsnummer: WO 2014/060161

(56) Entgegenhaltungen:
- EP-A1- 2 154 494
- EP-A1- 2 439 499
- EP-A1- 2 487 355
- WO-A1-2011/070535
- DE-A1-102008 029 793

## Beschreibung

### Stand der Technik

Aus dem Stand der Technik sind zahlreiche Verfahren und Vorrichtungen zur Bestimmung wenigstens einer Eigenschaft eines strömenden fluiden Mediums bekannt, also von Flüssigkeiten und/oder Gasen. Bei den Eigenschaften als möglichem Parameter kann es sich dabei grundsätzlich um beliebige physikalische und/oder chemisch messbare Eigenschaften handeln, welche eine Strömung des fluiden Mediums qualifizieren oder quantifizieren. Insbesondere kann es sich dabei um eine Strömungsgeschwindigkeit und/oder einen Massenstrom und/oder einen Volumenstrom handeln. Eine weitere Eigenschaft des strömenden fluiden Mediums ist der Feuchtegehalt.

Die Erfindung wird im Folgenden insbesondere beschrieben unter Bezugnahme auf so genannte Feuchtesensoren wie sie beispielsweise in Konrad Reif (Hrsg.): Sensoren im Kraftfahrzeug, 1. Auflage 2010, S. 98-101, beschrieben sind.

Ein derartiger Feuchtesensor kann in einem Gehäuse einer eigenen Sensoranordnung oder in einem Gehäuse eines so genannten Heißfilmluftmassenmessers angeordnet sein. Eine derartige Anordnung ist beispielsweise in der DE 10 2010 043 062 A1 beschrieben. Der Feuchtesensor ist dabei in dem Sensorgehäuse des Heißfilmluftmassenmessers untergebracht. Das Sensorgehäuse weist eine Einlassöffnung für Feuchtigkeit auf, die von einer semipermeablen Membran verschlossen ist. Die Feuchtigkeit durchdringt die Membran und gelangt so zu dem Feuchtesensor im Inneren des Sensorgehäuses. Die Membran ist dabei vorgesehen, um Schmutzpartikel zurückzuhalten.

Andere Anordnungen sind beispielsweise in EP 2 487 355 A1, DE 10 2008 029793 A1 und WO 2011/070535 A1 beschrieben.

Trotz der zahlreichen Vorteile der aus dem Stand der Technik bekannten Verfahren und Vorrichtungen zum Erfassen des Feuchtegehalts beinhalten diese noch Verbesserungspotential. So besteht bei den obigen Anordnungen die Gefahr, dass die Membran des Feuchtesensors im Betrieb durch beispielsweise Wasser, Staub oder Ölnebel verschmutzt werden kann. Dadurch kann weniger Feuchtigkeit als bei einer unverschmutzten Membran zu dem Feuchtesensor gelangen. Dies kann zu Fehlern in der Bestimmung der Feuchtegehalts führen.

### Offenbarung der Erfindung

Es wird daher eine Sensoranordnung zur Bestimmung eines Feuchtegehalts eines in einer Hauptströmungsrichtung strömenden fluiden Mediums vorgeschlagen, welche die Nachteile bekannter Verfahren und Strategien zumindest weitgehend vermeiden kann und bei der die Gefahr einer Verschmutzung einer Membran vermieden werden kann, wodurch die Genauigkeit der Bestimmung des Feuchtegehalts verbessert wird.

Die Sensoranordnung zur Bestimmung eines Feuchtegehalts eines in einer Hauptströmungsrichtung strömenden fluiden Mediums, insbesondere einer Ansaugluft einer Brennkraftmaschine, umfasst ein Sensorgehäuse, insbesondere einen in ein Strömungsrohr eingebrachten oder einbringbaren Steckfühler, mindestens einen in dem Sensorgehäuse angeordneten Feuchtesensor zur Bestimmung des Feuchtegehalts des fluiden Mediums und mindestens ein Rückhalteelement, wobei das Rückhalteelement für Feuchtigkeit zumindest teilweise durchlässig ist, wobei die Sensoranordnung eine Einlassöffnung für Feuchtigkeit in das Sensorgehäuse aufweist und der Feuchtesensor und das Rückhalteelement so in dem Sensorgehäuse angeordnet sind, dass der Feuchtesensor über die Einlassöffnung und das Rückhalteelement mit Feuchtigkeit beaufschlagbar ist, wobei die Sensoranordnung mindestens eine von der Einlassöffnung getrennt angeordnete Auslassöffnung für Feuchtigkeit aus dem Sensorgehäuse in das strömende fluide Medium aufweist, wobei das Sensorgehäuse so ausgebildet ist, dass das fluide Medium im Bereich der Auslassöffnung schneller strömt als im Bereich der Einlassöffnung, wobei das Sensorgehäuse im Wesentlichen die Form eines Tragflächenprofils aufweist, wobei die Einlassöffnung und die Auslassöffnung auf einer Oberseite des Tragflächenprofils angeordnet sind.

Die Einlassöffnung kann sich in der Hauptströmungsrichtung gesehen stromabwärts der Auslassöffnung befinden. Das Rückhalteelement kann den Feuchtesensor zumindest teilweise überspannen. Das Rückhalteelement kann zwischen der Einlassöffnung und der Auslassöffnung von dem fluiden Medium entgegen der Hauptströmungsrichtung überströmbar sein. Der Feuchtesensor kann eine Messoberfläche aufweisen, wobei das Sensorgehäuse so ausgebildet ist, dass innerhalb des Sensorgehäuses zwischen der Einlassöffnung und der Auslassöffnung die Messoberfläche des Feuchtesensors von dem fluiden Medium parallel überströmbar ist. Das Sensorgehäuse kann einen Kanal aufweisen, wobei der Kanal zwischen der Einlassöffnung und der Auslassöffnung angeordnet ist, wobei der Feuchtesensor in dem Kanal angeordnet ist. Das Sensorgehäuse kann eine Anströmkante aufweisen, die dem fluiden Medium entgegenweist. Das Sensorgehäuse ist so ausgebildet, dass das fluide Medium im Bereich der Auslassöffnung schneller strömt als im Bereich der Einlassöffnung. Erfindungsgemäß weist das Sensorgehäuse im Wesentlichen die Form eines Tragflächenprofils auf, wobei die Einlassöffnung und die Auslassöffnung auf einer Oberseite des Tragflächenprofils angeordnet sind. Das Sensorgehäuse ist so ausgebildet, dass das fluide Medium im Bereich der Einlassöffnung einen höheren statischen Druck als im Bereich der Auslassöffnung aufweist. Das Sensorgehäuse kann zumindest zwischen Einlass und Auslass im Wesentlichen die Form eines Tragflächenprofils aufweisen, wobei die Einlassöffnung und die Auslassöffnung beispielsweise stromabwärts bzw. stromaufwärts des Tragflächenprofils angeordnet sind. Die beispielsweise tragflächenförmig ausgebildete Strömungskontur zwischen der Einlassöffnung und der Auslassöffnung hat hauptsächlich die Aufgaben, eine für die Durchströmung des Kanals zwischen der Einlassöffnung und der Auslassöffnung möglichst große Druckdifferenz zu erzeugen sowie eine Fliehkraft-Abscheidung für Partikel und Tröpfchen zu realisieren. Dabei sind die Gegebenheiten der gesamten Sensoranordnung, insbesondere die Abmessungen des Sensorgehäuses zu berücksichtigen. Eine Abweichung von in der Luftfahrt üblichen Tragflächenprofilen besteht beispielsweise darin, dass im Bereich der Einlassöffnung bewusst eine Zone hohen statischen Druckes erzeugt wird und sich in diesem Bereich zielgerichtet keine an der Kontur anliegende Strömung ausbildet. Im Bereich der Auslassöffnung soll hingegen ein möglichst niedriger statischer Druck bei dementsprechend hohen Strömungsgeschwindigkeiten erzeugt werden. Daher bezieht sich die Angabe der Tragflächenform für den Bereich zwischen der Einlassöffnung und der Auslassöffnung auf die Beschreibung der körperlichen Form oder das äußere Aussehen, nicht aber auf die Funktionsweise, wie sie aus der Aerodynamik bekannt ist. Die Einlassöffnung und/oder die Auslassöffnung können im Wesentlichen schlitzförmig ausgebildet sein. In dem Sensorgehäuse kann ein Bypasskanal ausgebildet sein, wobei das Sensorgehäuse einen Einlass in den Bypasskanal, der einer Hauptströmungsrichtung des fluiden Mediums entgegenweist, und mindestens einen Auslass aus dem Bypasskanal des Sensorgehäuses aufweisen, wobei die Sensoranordnung ferner einen weiteren Sensor zur Bestimmung wenigstens eines weiteren Parameters eines durch den Bypasskanal strömenden fluiden Mediums, insbesondere einer Ansaugluftmasse einer Brennkraftmaschine, aufweisen kann, wobei der Sensor mindestens einen in dem Bypasskanal angeordneten Sensorchip zur Bestimmung des weiteren Parameters des fluiden Mediums aufweist.

Unter der Hauptströmungsrichtung ist im Rahmen der vorliegenden Erfindung die lokale Strömungsrichtung des fluiden Mediums am Ort des Sensors bzw. der Sensoranordnung zu verstehen, wobei beispielsweise lokale Unregelmäßigkeiten wie z. B. Turbulenzen unberücksichtigt bleiben können. Insbesondere kann unter der Hauptströmungsrichtung somit die lokale gemittelte Transportrichtung des strömenden fluiden Mediums am Ort der Sensoranordnung verstanden werden. Dabei bezieht sich die gemittelte Transportrichtung auf eine Transportrichtung, in der das fluide Medium im zeitlichen Mittel überwiegend strömt.

Unter einem Feuchtesensor ist im Rahmen der vorliegenden Erfindung ein beliebiges Sensorelement zu verstehen, welches eingerichtet ist, um eine Feuchtigkeit des fluiden Mediums zu erfassen. Beispielsweise kommen hierbei resistive und/oder kapazitive Sensorelemente in Betracht, wie sie aus dem Stand der Technik bekannt sind. Beispiele derartiger Feuchtesensoren sind in Konrad Reif, (Hrsg.): Sensoren im Kraftfahrzeug, 1. Auflage 2010, S. 98-101, beschrieben. Auch andere Arten von Feuchtesensoren kommen jedoch grundsätzlich alternativ oder zusätzlich für den Einsatz im Rahmen der vorliegenden Erfindung in Betracht. Der Feuchtegehalt kann dabei als absoluter Wert in beispielsweise Gramm Wasser pro Kilogramm oder Kubikmeter Luft ausgedrückt werden. Alternativ oder zusätzlich kann der Feuchtegehalt als relative Luftfeuchtigkeit in Prozent ausgedrückt werden. Unter der relativen Luftfeuchtigkeit ist im Rahmen der vorliegenden Erfindung das prozentuale Verhältnis zwischen dem momentanen Dampfdruck des Wassers und dem Sättigungsdampfdruck desselben bei der Lufttemperatur zu verstehen. Die relative Feuchtigkeit lässt unmittelbar erkennen, in welchem Grad die Luft mit Wasserdampf gesättigt ist.

Unter einem Rückhalteelement ist im Rahmen der vorliegenden Erfindung ein beliebiges Element zu verstehen, das eingerichtet ist, zumindest gröbere Verunreinigungen wie Staubpartikel, Schmutz, Flüssigkeitstropfen oder ähnliche größere Verunreinigungen, beispielsweise mit einer Größe von mehr als 0,2 mm, vorzugsweise von mehr als 0,5 mm, zurückzuhalten, wohingegen Feuchtigkeit des fluiden Mediums, beispielsweise Luftfeuchtigkeit, das Rückhalteelement durchdringen kann. Das Rückhalteelement kann insbesondere mindestens eine feuchtigkeitsdurchlässige Membran, beispielsweise eine Kunststoffmembran und/oder mindestens ein Gewebe, beispielsweise mindestens ein Netz, umfassen.

Unter der Angabe "im Bereich" für eine Position ist im Rahmen der vorliegenden Erfindung eine Position zu verstehen, die sich innerhalb einer Ebene senkrecht zu der Hauptströmungsrichtung erstreckt und das angegebene Bezugsbauteil enthält. Beispielsweise bedeutet die Angabe, dass das fluide Medium im Bereich der Einlassöffnung einen bestimmten Druck aufweist, eine Position, an der der Druck bestimmt wird, die sich innerhalb einer Ebene befindet, die senkrecht zu der Hauptströmungsrichtung verläuft und die Einlassöffnung bzw. eine effektive Fläche der Einlassöffnung enthält. Unter einer effektiven Fläche der Einlassöffnung ist die Fläche der Einlassöffnung selbst und ihre unmittelbare Umgebung zu verstehen. Dadurch treibt im Bereich der Einlassöffnung ein effektiver, relativ hoher gemittelter statischer Druck in Verbindung mit einem im Bereich der Auslassöffnung effektiven, relativ niedrigen gemitteltem statischen Druck die Durchströmung durch den Kanal.

Unter der Angabe "stromabwärts" ist im Rahmen der vorliegenden Erfindung eine Anordnung zu verstehen, bei der das in Verbindung mit dieser Angabe genannte Bauteil von dem in der Hauptströmungsrichtung strömenden fluiden Medium zu einem späteren Zeitpunkt erreicht wird als ein Bezugsbauteil. Beispielsweise bedeutet die Angabe, dass sich die Einlassöffnung stromabwärts der Auslassöffnung befindet, dass das in der Hauptströmungsrichtung strömende fluide Medium zeitlich gesehen zuerst die Auslassöffnung und dann die Einlassöffnung erreicht.

Unter der Angabe "stromaufwärts" ist im Rahmen der vorliegenden Erfindung eine Anordnung zu verstehen, bei der das in Verbindung mit dieser Angabe genannte Bauteil von dem in der Hauptströmungsrichtung strömenden fluiden Medium zu einem früheren Zeitpunkt erreicht wird als ein Bezugsbauteil. Beispielsweise bedeutet die Angabe, dass sich die Auslassöffnung stromaufwärts der Einlassöffnung befindet, dass das in der Hauptströmungsrichtung strömende fluide Medium zeitlich gesehen zuerst die Auslassöffnung und dann die Einlassöffnung erreicht.

Unter der Form eines Tragflächenprofils ist im Rahmen der vorliegenden Erfindung eine Form zu verstehen, die in einer Seitenansicht gesehen näherungsweise die Form einer Tragfläche aufweist, d. h. mit in Hauptströmungsrichtung gegenüberliegenden gewölbten Seitenflächen. Die Seitenflächen können dabei eine unterschiedliche Krümmung oder Wölbung aufweisen. Quer zur Hauptströmungsrichtung sind veränderliche oder unveränderliche Konturen denkbar. Die Kontur der stromabwärts liegenden Seitenfläche, welche sich also unmittelbar stromaufwärts der Einlassöffnung befindet, kann so gestaltet sein, dass die Strömung dort ablöst, was durch eine deutlich, aber stetig, also sprungfrei bezüglich der Krümmung zurückgenommene Kontur erreicht wird. Eine in der Strömungsmechanik-Literatur als zurückspringende Stufe bezeichnete Kontur, also eine sich sprunghaft ändernde Kontur, bei der sich an der Sprungstelle der Strömungsquerschnitt abrupt vergrößert, ist ebenfalls denkbar. Allerdings entsteht an solch einer zurückspringenden Stufe eine geometrisch induzierte Ablösung mit einer stromab recht dünnen Strömungsscherschicht und einer recht stabilen Rezirkulationszone, in die nur relativ wenig Fluid der Außenströmung eingemischt wird. Der zu messende Feuchtegehalt der Außenströmung liegt also nicht sofort im Bereich der Einlassöffnung an. Günstiger in dieser Hinsicht sollte eine stärkere Durchmischung sein, ohne jedoch Fluid- oder Feststoffpartikel in den Einlass zu befördern.

Unter dem Druck ist im Rahmen der vorliegenden Erfindung der Betrag einer auf eine Fläche normal stehenden Kraft je Flächeninhalt der Fläche zu verstehen. Der Druck in strömenden Medien setzt sich aus einem statischen und einem dynamischen Anteil zusammen. Während beide Teile von der Dichte abhängen, unterscheiden sie sich dadurch, dass der statische Druck, für Fluide mit konstanter Dichte, linear mit der Höhe der Fluidsäule über der Fläche steigt. Zudem ist er von der Erdbeschleunigung, also der Gravitation, abhängig. Der dynamische Anteil hingegen wächst quadratisch mit der Strömungsgeschwindigkeit des Fluids. In einer reibungsfreien Strömung besteht eine Konstanz der Summe aus dynamischem und statischem Anteil. Dieses ist die Konsequenz aus der Energieerhaltung in der Strömung und für diesen Spezialfall als Gesetz von Bernoulli bekannt.

Im Rahmen der vorliegenden Erfindung ist unter dem statischen Druck das Verhältnis einer auf jede Fläche, die mit dem Fluid in Verbindung steht, ausgeübten Kraft zu verstehen, die zur Größe der Fläche proportional wirkt. Der statische Druck lässt sich beispielsweise anhand der Gleichung Pstat. = ρ ^{∗} g ^{∗} h ermitteln, wobei ρ die Dichte des Fluids, g die Erdbeschleunigung und h die Höhe der Fluidsäule über der Fläche ist.

Im Rahmen der vorliegenden Erfindung ist unter dem dynamischen Druck der aus der kinetischen Energie eines strömenden Fluids an der Oberfläche eines Körpers in dieser Strömung resultierende Wert zu verstehen. Der dynamische Druck lässt sich anhand der Gleichung Pdyn. = 0,5 ^{∗} ρ ^{∗} v² ermitteln, wobei ρ die Dichte des Fluids und v die Geschwindigkeit des Fluids ist. Der dynamische Druck nimmt somit zusammen mit der Geschwindigkeit des Fluids zu und ab. Die Geschwindigkeit kann von größeren zu kleineren Querschnittsflächen aber nur zunehmen, wenn der statische Druck in den kleineren Querschnittsflächen niedriger ist und umgekehrt. Durch die Strömung entsteht also beim Übergang von einer größeren in eine kleinere Querschnittsfläche eine dynamische Verringerung des statischen Druckes bei gleichzeitiger Erhöhung des dynamischen Druckes. Umgekehrt erhöht sich der statische Druck beim Übergang von einer kleineren zu einer größeren Querschnittsfläche, während der dynamische Druck sinkt. Der dynamische Druck ist dabei zwar in der Regel nicht direkt messbar, wird aber zur Geschwindigkeitsmessung des Fluids verwendet.

Im Rahmen der vorliegenden Erfindung kann die Sensoranordnung einen alleinigen Feuchtesensor umfassen. Es ist jedoch ebenfalls möglich, dass weitere Sensoren von der Sensoranordnung umfasst werden, wie beispielsweise einen Heißfilmluftmassenmesser und/oder einen Temperatursensor und/oder einen Drucksensor.

Im Rahmen der vorliegenden Erfindung kann der Feuchtesensor beispielsweise in einem Gehäuse eines Heißfilmluftmassenmessers integriert sein, wie sie beispielsweise in Konrad Reif (Hrsg.): Sensoren im Kraftfahrzeug, 1. Auflage 2010, Seite, 146-148 beschrieben sind. Derartige Heißfilmluftmassenmesser basieren in der Regel auf einem Sensorchip, insbesondere einem Silizium-Sensorchip, beispielsweise mit einer Sensormembran als Messoberfläche oder Sensorbereich, welche von dem strömenden fluiden Medium überströmbar ist. Der Sensorchip umfasst in der Regel mindestens ein Heizelement sowie mindestens zwei Temperaturfühler, welche beispielsweise auf der Messoberfläche des Sensorchips angeordnet sind, wobei ein Temperaturfühler stromaufwärts des Heizelements und der andere Temperaturfühler stromabwärts des Heizelements gelagert ist. Aus einer Asymmetrie des von den Temperaturfühlern erfassten Temperaturprofils, welches durch die Strömung des fluiden Mediums beeinflusst wird, kann auf einen Massenstrom und/oder Volumenstrom des fluiden Mediums geschlossen werden.

Heißfilmluftmassenmesser sind üblicherweise als Steckfühler ausgestaltet, welcher fest oder austauschbar in ein Strömungsrohr einbringbar sind. Beispielsweise kann es sich bei diesem Strömungsrohr um einen Ansaugtrakt einer Brennkraftmaschine handeln.

Durch die Erfindung wird das Rückhalteelement, das beispielsweise in Form einer Schutzmembran ausgebildet ist, vor direktem Kontakt mit Partikeln und Tröpfchen geschützt, wobei gleichzeitig eine ausreichende Überströmung mit Luft gewährleistet wird.

So kann die Feuchte richtig gemessen werden und die Funktion des Feuchtesensors ist auch in einer Umgebung, die stark mit beispielsweise Partikeln, Wassertropfen oder Öldampf kontaminiert ist, sichergestellt. Dies kann durch die spezielle strömungstechnische Anordnung von Ein- und Auslassschlitzen auf dem ansonsten geschlossenen Deckel des Sensorgehäuses in Kombination mit einem speziellen Profil, das am Einlass Überdruck und am Auslass Unterdruck erzeugt, erreicht werden. Entsprechend werden die funktionsbestimmenden Elemente des Sensorgehäuses, wie beispielsweise die Anströmkante des Steckfühlers, sowie der Einlassschlitz, der Auslassschlitz und die Strömungskontur des Deckels derart gestaltet, dass die Schutzmembran nicht mehr direkt dem fluiden Medium ausgesetzt ist. Bei richtiger Ausführung der Strömungskontur des Deckels ergeben sich Strömungsverhältnisse bzw. Druckverhältnisse an der Schutzmembran des Feuchtesensors, die derart sind, dass sich der Einlassschlitz in einer Zone befindet, in der die Strömung verlangsamt wird bzw. eine geringe Strömungsgeschwindigkeit und/oder die Grenzschicht abgelöst ist und damit ein relativ hoher statischer Druck vorliegt, die Auslassöffnung sich in einer Zone befindet, in der die Strömung beschleunigt wird bzw. eine hohe Strömungsgeschwindigkeit vorliegt. Aufgrund der strömungsfeld-definierenden Wirkung des Sensorgehäuses und des Deckels mit dem speziellen Profil ergibt sich eine günstige Anordnung mit dem Einlassschlitz im stromabwärtigen und dem Auslassschlitz des stromaufwärtigen Teils des Deckels. Damit wird die Schutzmembran entgegen der Hauptströmungsrichtung durchströmt.

Somit ist die Zone, in der die Luft eingesaugt wird, weitgehend frei von Partikeln oder Tröpfchen, da diese aufgrund ihrer Trägheit die Zone mit niedriger Luftgeschwindigkeit durchqueren bzw. gar nicht erreichen und weiter in der Hauptströmungsrichtung fliegen. Somit ist die Luft, die an der Schutzmembran des Sensorelements vorbeigeleitet wird, weitgehend befreit von Partikeln und Tröpfchen und eine Kontamination wird dadurch vermieden. Ein wesentliches Element zur Erzeugung der Durchströmung ist die Deckelkontur in Verbindung mit der Anströmkante des Steckfühlers. Eine geeignete Gestaltung ist dadurch gekennzeichnet, dass die Strömung am Einlassschlitz verlangsamt und am Auslassschlitz beschleunigt wird. Dies kann durch ein tragflächenähnliches Profil erreicht werden. Beispielsweise kann die Strömungskontur alleine durch den Deckel gestaltet werden. Alternativ kann die Anströmkante, also die stromaufwärtige Stirnfläche des Sensorgehäuses in die Gestaltung miteinbezogen werden. Denkbar ist ebenfalls, dass eine von Flügelprofil abgeleitete Form vorgesehen wird. Diese kann durch Schlote am Auslass weiter verbessert werden. Denkbar ist jedoch auch eine kantigere Ausführung, die konstruktiv leichter umzusetzen sein kann. Die Kontur im Innenbereich, d. h. im Inneren des Sensorgehäuses, insbesondere in unmittelbarer Nähe des Messelements, ist ein weiterer Aspekt, der zu beachten ist. Durch die vorteilhafte Gestaltung der Innenkontur ergibt sich ein möglichst hoher Massenstrom in unmittelbarer Nähe, also in der wandnahen Strömungsschicht des Messelements. Dieser möglichst große Luftmassenstrom wird angestrebt, um mit einer möglichst geringen Verzögerung, also einer möglichst kleinen Zeitdauer bis zur geforderten Größe der Signal-Sprungantwort die tatsächlich vorhandene Luftfeuchte bestimmen zu können.

Um dies zu erreichen, sollte die Luft möglichst parallel zur Oberfläche des Messelements geführt werden. Ablösungen an der Vorderkante des Messelements bezogen auf die Durchströmung im Innenbereich sowie Rückström- oder Totwassergebiete oberhalb des Messelements sollten vermieden werden. Solche Rückström- oder Totwassergebiete können eine zirkulierende Luftmasse enthalten, deren Feuchte nicht der aktuell vorhandenen, zu messenden Feuchte entspricht. Es ergeben sich durch solche Gebiete unnötige Totzeiten bezüglich der Signalwerte. Entsprechende Varianten der Innenkontur des Deckels verlaufen nicht parallel zur Außenkontur, sondern eher parallel zum Messelement. Der sich ergebende Kanal zwischen Messelement und Deckel darf wiederum nicht zu klein sein, weil sich ansonsten ein hoher Durchströmungswiderstand ergeben würde.

Des Weiteren kann es vorteilhaft sein, die Vorderkante am Messelement strömungsgünstig zu gestalten, also beispielsweise abgerundet oder durch eine angespritzte Schräge, um eine Ablösung und ein beschriebenes Rückströmungsgebiet zu vermeiden. Die Vorderkante ist dabei diejenige Kante die dem durch die Einlassöffnung in das Innere des Sensorgehäuses zuweisenden Teilstrom des fluiden Mediums zugewandt ist.

### Kurze Beschreibung der Zeichnungen

Weitere optionale Einzelheiten und Merkmale der Erfindung ergeben sich aus der nachfolgenden Beschreibung bevorzugter Ausführungsbeispiele, welche in den Figuren schematisch dargestellt sind.

Es zeigen:
- Figur 1: eine perspektivische Darstellung einer Sensoranordnung,
- Figur 2: eine Querschnittsansicht der Sensoranordnung,
- Figur 3: eine schematische Darstellung der Verteilung der Strömungsgeschwindigkeit bei Umströmung der Sensoranordnung,
- Figur 4: eine schematische Darstellung der Druckverteilung bei Umströmung der Sensoranordnung,
- Figur 5A: Darstellung einer weiteren Sensoranordnung, und
- Figur 5B-5D: Darstellungen erfindungsgemäßer Ausführungsformen der Sensoranordnung, und
- Figur 6: eine Unteransicht eines Deckels.

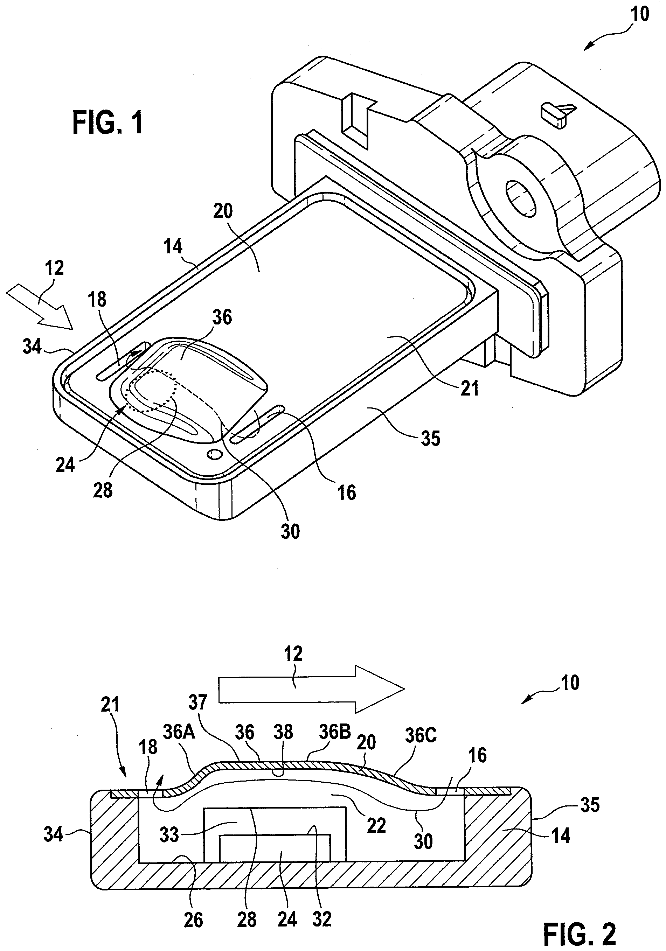

Figur 1 zeigt eine erste Ausführungsform einer Sensoranordnung 10 zur Bestimmung eines Feuchtegehalts eines in einer Hauptströmungsrichtung 12 strömenden fluiden Mediums, insbesondere einer Ansaugluft einer Brennkraftmaschine. Bei diesem Ausführungsbeispiel umfasst die Sensoranordnung 10 ein Sensorgehäuse 14, welches beispielsweise als ein Steckfühler ausgebildet sein kann, der in ein Strömungsrohr, insbesondere einen Ansaugtrakt der Brennkraftmaschine, eingesteckt oder eingebracht werden kann. Die Sensoranordnung 10 umfasst eine Einlassöffnung 16 und eine Auslassöffnung 18 in das bzw. aus dem Sensorgehäuse 14. Die Einlassöffnung 16 und die Auslassöffnung 18 sind in einem Deckel 20 des Sensorgehäuses 14 ausgebildet. In dem gezeigten Ausführungsbeispiel befinden sich die Einlassöffnung 16 und die Auslassöffnung 18 in dem Deckel 20 auf einer Seite, die sich parallel zu der Hauptströmungsrichtung 12 und einer Längserstreckungsrichtung des Sensorgehäuses 14 erstreckt, die bezogen auf die Darstellung in Figur 1 beispielsweise eine Oberseite 21 ist. Die Oberseite 21 ist beispielsweise eine Seite, die sich senkrecht zu einer der Hauptströmungsrichtung 12 zuweisenden Seite erstreckt. Der Deckel 20 ist somit auf der Oberseite 21 des Sensorgehäuses 14 angeordnet bzw. definiert diese. In einem in das Sensorgehäuse 14 eingesetzten Zustand des Deckels 20 befindet sich die Einlassöffnung 16 in der Hauptströmungsrichtung 12 gesehen stromabwärts der Auslassöffnung 18. Beispielsweise sind die Einlassöffnung 16 und die Auslassöffnung 18 schlitzförmig ausgebildet, wobei die Abmessungen des schlitzförmigen Öffnungsquerschnitts in der Hauptströmungsrichtung 12 gesehen jeweils beispielsweise 1,5 mm betragen können. Die Schlitze können abgerundete oder scharfe Kanten auf der Innen- und/oder Außenseite aufweisen.

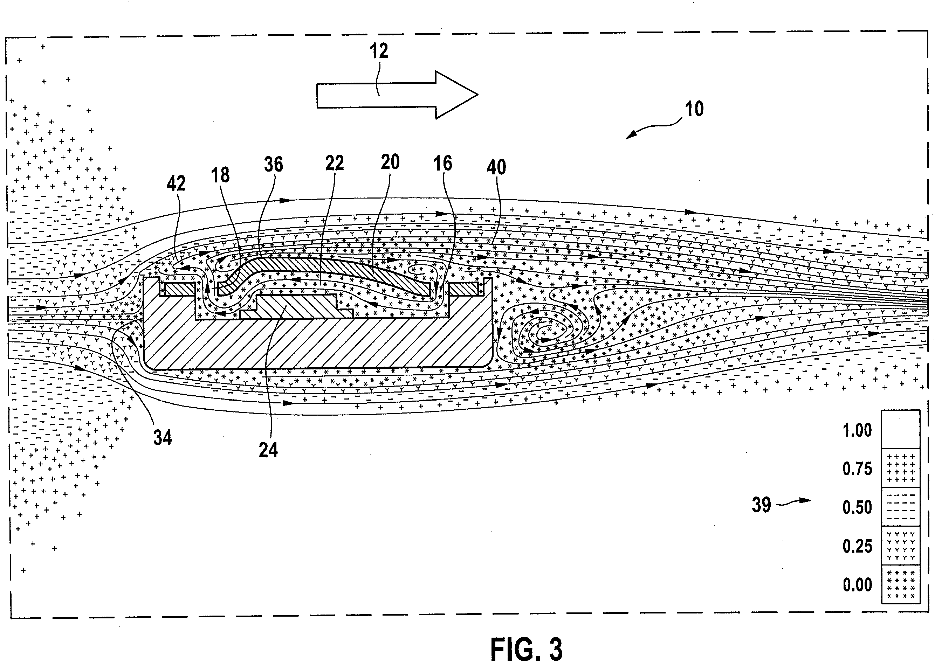

Wie der Figur 2 oder der Figur 3 zu entnehmen ist, ist im Inneren des Sensorgehäuses 14 zwischen der Einlassöffnung 16 und der Auslassöffnung 18 ein Kanal 22 gebildet. Im Inneren des Sensorgehäuses 12 ist ein Feuchtesensor 24 angeordnet. Der Feuchtesensor 24 ist in dem Kanal 22 angeordnet. Der Feuchtesensor 24 ist von dem Deckel 20 beabstandet. Beispielsweise ist der Feuchtesensor 24 auf einer dem Deckel 20 gegenüberliegenden Wand 26 des Kanals 22 angeordnet. Der Feuchtesensor 24 wird von einem Rückhalteelement 28, wie beispielsweise einer Membran, überspannt. Figur 2 zeigt beispielsweise, dass der Feuchtesensor 24 innerhalb des Sensorgehäuses 14 in einer der Hauptströmungsrichtung 12 entgegengesetzten Strömungsrichtung 30 überströmt wird. Insbesondere weist der Feuchtesensor 24 eine Messoberfläche 32 auf, die dem Kanal 22 zugewandt ist. Der Feuchtesensor 24 ist so angeordnet, dass das fluide Medium seine Messoberfläche 32 möglichst parallel überströmen kann. Zwischen dem Feuchtesensor 24 und und von dem Rückhalteelement 28 ist ein kleiner Spalt 33 vorgesehen. Der Spalt 33 ist insbesondere zwischen der Messoberfläche 32 und dem Rückhalteelement 28 gebildet. Der Spalt 33 kann jedoch auch an den Seitenflächen des Feuchtesensors 24 vorgesehen, d.h. bezogen auf die Darstellung der Figur 2 links und rechts von dem Feuchtesensor 24.

Das Sensorgehäuse 14 weist eine Anströmkante 34 auf, die dem in der Hauptströmungsrichtung 12 strömenden fluiden Medium entgegenweist und eine Stirnfläche des Sensorgehäuses 14 bildet. Beispielsweise erstreckt sich die Anströmkante 34 senkrecht zu der Hauptströmungsrichtung 12. Ferner weist das Sensorgehäuse 14 an seinem in der Hauptströmungsrichtung 12 gesehen stromabwärtigen Ende eine Hinterkante 35 auf, die sich senkrecht zu der Hauptströmungsrichtung 12 erstreckt. Der Deckel 20 umfasst beispielsweise zumindest einen Bereich 36 zwischen der Einlassöffnung 16 und der Auslassöffnung 18, der näherungsweise in Form eines Tragflächenprofils ausgebildet ist. Der Bereich 36 wird somit in der Hauptströmungsrichtung 12 gesehen von einem Anströmbereich 36A, der ansteigt und sich somit von dem Sensorgehäuse 14 mit zunehmender Bewegung in der Hauptströmungsrichtung 12 entfernt, einem Überströmbereich 36B, der sich im Wesentlichen parallel zu der Haupströmungsrichtung 12 erstreckt und einem Abströmbereich 36 C, der in der Hauptströmngsrichtung 12 gesehen abfällt und sich somit dem Sensorgehäsue 14 mit zunehmender Bewegung in der Hauptströmungsrichtung 12 nähert, gebildet. Bezüglich der Hauptströmungsrichtung 12 kann der Deckel 20 stromaufwärts der Auslassöffnung 18 und stromabwärts der Einlassöffnung 16 parallel zu der Hauptströmungsrichtung 12 ausgebildet sein. Die Einlassöffnung 16 und die Auslassöffnung 18 sind dabei stromabwärts bzw. stromaufwärts des speziellen Profils des Bereichs 36 angeordnet. Der Bereich 36 weist somit eine von dem Sensorgehäuse 14 gewölbt vorstehende Oberseite 37, die dem in der Hauptströmungsrichtung 12 strömenden fluiden Medium ausgesetzt ist, und eine Unterseite 38 auf, wobei die Unterseite 38 des Bereichs 36 dem Kanal 22 zuweist und so dem in der Richtung des Pfeils 30 strömenden fluiden Medium ausgesetzt ist. Das spezielle Profil des Bereichs 36 zeichnet sich dadurch aus, dass es stromaufwärts an der Auslassöffnung 18 einen niedrigeren statischen Druck als stromabwärts an der Einlassöffnung 16 erzeugt und damit die Strömung in dem Kanal 22 ursächlich antreibt.

Durch die oben beschriebene Ausbildung bzw. Form der Sensoranordnung 10 ergibt sich die in Figur 3 dargestellte Verteilung der Strömungsgeschwindigkeit. In Figur 3 sind dabei Stromlinien und Strömungsbereiche eingezeichnet, denen bestimmte Strömungsgeschwindigkeiten zugeordnet sind. Die jeweiligen Strömungsgeschwindigkeiten sind in der Skala 39 der Figur 3 dargestellt, in der die Strömungsgeschwindigkeit in Meter pro Sekunde (m/s) aufgetragen ist. Aus diesem wird deutlich, dass sich die Einlassöffnung 16 in einem Strömungsbereich 40 befindet, in der die Strömung verlangsamt ist und/oder eine Strömungsgrenzschicht abgelöst ist und damit hohe statische Drücke vorliegen. Die Auslassöffnung 18 befindet sich in einem Strömungsbereich 42, in dem hohe Strömungsgeschwindigkeiten und damit niedrige statische Drücke vorliegen. So findet von dem Strömungsbereich 42 nahe der Anströmkante 34 an der Auslassöffnung 18 beispielsweise eine Verzögerung von ca. 0,75 m/s auf ca. 0,25 m/s in dem Strömungsbereich 40 der Einlassöffnung 16 statt.

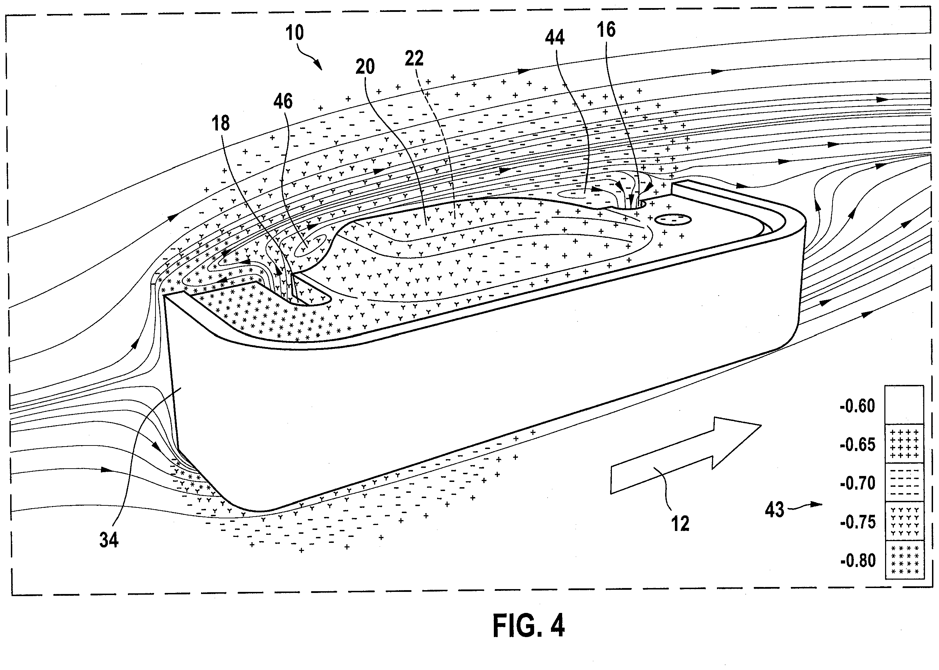

Figur 4 zeigt ferner die Verteilung des statischen Drucks in einem Querschnitt bei einer erfindungsgemäßen Ausbildung der Sensoranordnung 10. In Figur 4 sind dabei zusätzlich die Stromlinien eingezeichnet. Die Ausbildung der Stromlinien korrespondiert mit der flächigen Verteilung des Druckes. Die jeweiligen Drücke sind in der Skala 43 der Figur 4 dargestellt, in der der Druck in Pascal (Pa) aufgetragen ist. Es handelt sich hierbei um einen relativen Druck zum absoluten Umgebungsdruck von 1 bar. Bei Strömungssimulationen wird aus Gründen der numerischen Genauigkeit oft mit Relativdrücken gerechnet, die nachträglich auf der Basis eines bekannten Bezugsdruckes wieder zu einem Absolutdruck bestimmt werden können. Auch in diesem Fall liegt also ein positiver Absolutdruck vor. Da die Strömung aufgrund der Druckunterschiede durch den Kanal 22 getrieben wird, ist auch eine Relativbetrachtung wie in Figur 4 sinnvoll. Wie der Figur 4 zu entnehmen ist, befindet sich die Einlassöffnung 16 in einem Bereich 44, in dem der statische Druck höher ist als in dem Bereich 46, in dem sich die Auslassöffnung 18 befindet. Mit anderen Worten weist das strömende fluide Medium im Bereich der Einlassöffnung 16 einen höheren statischen Druck als im Bereich der Auslassöffnung 18 auf. Beispielsweise ist der statische Druck -0,75 Pa im Bereich der Auslassöffnung 18 und -0,65 Pa im Bereich der Einlassöffnung 16. Dadurch wird das fluide Medium in die Einlassöffnung 16 hineingedrückt und kann dann entgegen der Hauptströmungsrichtung 12 im Inneren des Sensorgehäuses 14 durch den Kanal 22 strömen, wobei der Feuchtesensor 24 durch das Rückhalteelement 28 von der in dem fluiden Medium enthaltenen Feuchtigkeit beaufschlagbar ist. Das fluide Medium strömt dann wiederum aus dem Kanal 22 durch die Auslassöffnung 18 in das in der Hauptströmungsrichtung 12 strömende fluide Medium. Die Einlassöffnung 16 ist dabei derart angeordnet, dass das fluide Medium weitgehend frei von Partikeln oder Tröpfchen ist, da diese aufgrund ihrer Trägheit den Bereich der Einlassöffnung 16 durchqueren bzw. gar nicht erst erreichen und weiter in der Hauptströmungsrichtung 12 fliegen und nicht in den Kanal 22 gelangen. Somit ist das fluide Medium, das an dem Rückhalteelement 28 des Feuchtesensors 24 vorbeigeleitet wird, weitestgehend befreit von Partikeln und Tröpfchen. Dadurch wird eine Kontamination des Rückhalteelements 24 vermieden und der Feuchtesensor 24 kann den Feuchtegehalt genau bestimmen.

Unter Rückkehr zur Darstellung der Figur 2 wird die in den Figuren 3 und 4 dargestellte Strömungsform durch den Deckel 20 in Verbindung mit der das Strömungsfeld mitdefinierenden Wirkung des Sensorgehäuses 14 gestaltet bzw. gebildet, der näherungsweise die Form eines Tragflächenprofils zwischen der Einlassöffnung 16 und der Auslassöffnung 18 aufweist. Aus Figur 2 wird dabei deutlich, dass das in der Hauptströmungsrichtung 12 strömende fluide Medium zuerst die Auslassöffnung 18 passiert und dann die Einlassöffnung 16 erreicht. Insbesondere ist durch den Pfeil 30 angedeutet, wie ein Teilstrom des fluiden Mediums durch die Einlassöffnung 16 in den Kanal 22 eindringt, in dem Kanal 22 parallel zu der Messoberfläche 32 des Feuchtesensors 24 und entgegen der Hauptströmungsrichtung 12 strömt, das Rückhalteelement 28 überstreicht und durch die Auslassöffnung 18 wieder in den Hauptstrom des fluiden Mediums gelangt. Während der Teilstrom in dem Kanal 22 das Rückhalteelement 28 überstreicht, kann Feuchtigkeit das Rückhalteelement 28 zu der Messoberfläche 32 durchdringen, so dass der Feuchtesensor 24 den Feuchtegehalt des Teilstroms bestimmen kann. Da der Feuchtegehalt des Teilstroms aufgrund der erfindungsgemäßen Gestaltung annähernd identisch bzw. nach einer gewissen Antwortzeit identisch mit dem Feuchtegehalt des Hauptstroms ist, kann der Feuchtesensor 24 so auf den Feuchtegehalt des in der Hauptströmungsrichtung 12 strömenden fluiden Mediums schließen und diesen bestimmen.

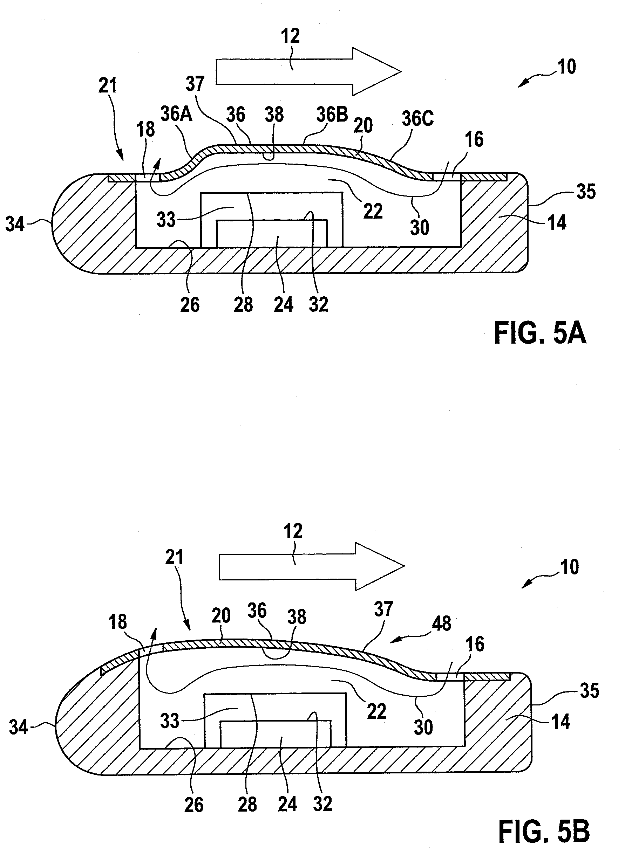

Weitere mögliche Ausführungsformen der Sensoranordnung 10 sind in den Figuren 5A bis 5D gezeigt. Nachfolgend werden nur die Unterschiede zu der ersten Ausführungsform beschrieben und gleiche Bauteile sind mit gleichen Bezugszeichen versehen.

In Figur 5A wird eine Anströmkante 34 des Sensorgehäuses 14 in die Gestaltung der in den Figuren 3 und 4 gezeigten Strömungsform mit einbezogen. Insbesondere ist die Anströmkante 34 gewölbt ausgebildet, wobei die Wölbung der Hauptströmungsrichtung 12 zugewandt ist, d.h. entgegen der Hauptströmungsrichtung 12 weist.

Figur 5B zeigt eine von einem Flügelprofil abgeleitete erfindungsgemäße Form des Sensorgehäuses 14, d.h. die Strömungsform wird nicht nur durch die Form des Deckels 20 zwischen der Einlassöffnung 16 und der Auslassöffnung 18, sondern durch die Form des gesamten Sensorgehäuses 14 und Deckels 20 beeinflusst. Insbesondere ist die Anströmkante 34 gewölbt ausgebildet, wobei die Wölbung der Hauptströmungsrichtung 12 zugewandt ist, d.h. entgegen der Hauptströmungsrichtung 12 weist. Ferner ist der gesamte Deckel 20 gewölbt, so dass das Sensorgehäuse 14 in der Form eines Tragflächenprofils 48 ausgebildet ist.

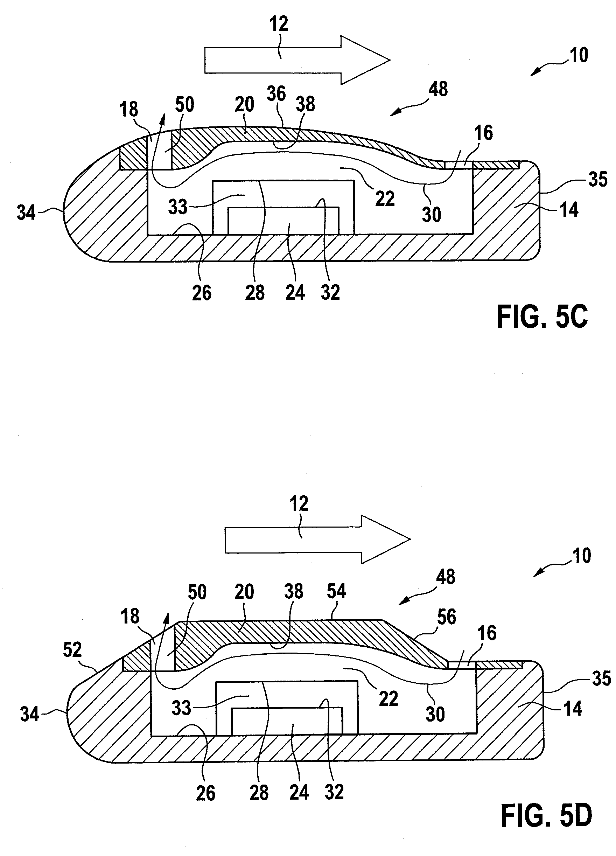

Figur 5C zeigt eine weitere erfindungsgemäße Ausführungsform, bei der die die Strömungsform durch eine kaminartige Verlängerung 50 im Bereich der Auslassöffnung 18 insbesondere im Innenbereich, also im Kanal 22 gesteuert wird. Durch entsprechende Konturierung der Verlängerung 50 kann die Überströmung des Rückhalteelementes 28 gesteuert werden.

Figur 5D zeigt schließlich eine erfindungsgemäße kantigere Ausführungsform des Sensorgehäuses 14 im Vergleich zu dem Sensorgehäuse 14 der Figur 5C, die konstruktiv leichter umzusetzen sein kann. Insbesondere ist der Deckel 20 so ausgebildet, dass er stromabwärts der Anströmkante 34 einen rampenförmig bis zu der Auslassöffnung 18 vorstehenden Abschnitt 52, dann stromabwärts der Auslassöffnung 18 einen parallel zur Hauptströmungsrichtung 12 verlaufenden Abschnitt 54, und weiter stromabwärts einen Abschnitt 56 aufweist, in Richtung zu der Einlassöffnung 16 abfällt, d.h. geneigt ist.

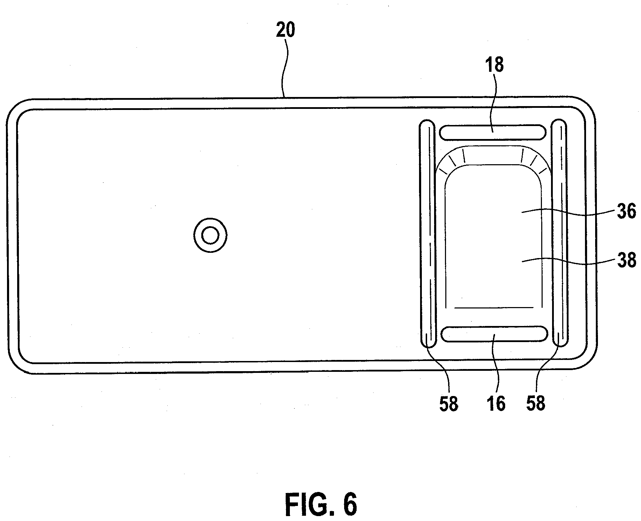

Figur 6 zeigt eine Unteransicht eines Beispiels für den Deckel 20. Gut zu erkennen ist die Form des Bereichs 36 zwischen der Einlassöffnung 16 und der Auslassöffnung 18. Aufgrund der Darstellung des Deckels 20 in einer Unteransicht ist der Bereich 36 dabei als Vertiefung zu sehen und man blickt auf die Unterseite 38. Denkbar ist auch eine von der Oberseite 36 abweichende Profilierung der Unterseite 38 aufgrund einer in diesem Bereich unterschiedlichen Dicke bzw. Materialstärke der Wand. Ferner sind aus der Darstellung der Figur 6 Seitenwände 58 des Kanals 22 zu erkennen, die den Kanal 22 begrenzen. Die Seitenwände 58 verlaufen beispielsweise parallel zueinander, wie in Figur 6 gezeigt. Die Seitenwände 58 können sich senkrecht zu der Einlassöffnung 16 und der Auslassöffnung 18 erstrecken. Dadurch erstrecken sich die Seitenwände 58 parallel zu dem in der Richtung des Pfeils 30 strömenden fluiden Mediums. Die Seitenwände 58 können beispielsweise rippenförmig von einer Unterseite des Deckels 20 vorstehen. Die in der Darstellung der Figur 6 zu sehenden Seitenwände 58 des Kanals 22 sorgen für eine Strömungsführung über das Rückhalteelement 28 und verhindern die Durchströmung des Elektronikmodulbereiches und eventuelle Rezirkulationsgebiete in diesem.

Alternativ zu der in Figur 6 gezeigten parallelen Ausbildung der Seitenwände 58 sind auch konvergente Seitenwände 58 in dem Kanal 22 möglich, die die Strömung von der Einlassöffnung 16 bis zu dem Rückhalteelement 28 beschleunigen und dann bis zur Auslassöffnung 18 wieder verzögern, um in dem Kanal 22 nach einem Überströmen des Rückhalteelements 28 unnötig hohe Wandreibungsverluste zu vermeiden.

Alle oben beschriebenen Ausführungsformen lassen sich in Verbindung mit weiteren Sensoren realisieren. Beispielsweise ist der Feuchtesensor in einem Sensorgehäuse eines Heißfilmluftmassenmessers der oben beschriebenen Art ausgebildet.

## Patentansprüche

1. Sensoranordnung (10) zur Bestimmung eines Feuchtegehalts eines in einer Hauptströmungsrichtung (12) strömenden fluiden Mediums, insbesondere einer Ansaugluft einer Brennkraftmaschine, wobei die Sensoranordnung (10) ein Sensorgehäuse (14), insbesondere einen in ein Strömungsrohr eingebrachten oder einbringbaren Steckfühler, mindestens einen in dem Sensorgehäuse (14) angeordneten Feuchtesensor (24) zur Bestimmung des Feuchtegehalts des fluiden Mediums und mindestens ein Rückhalteelement (28) aufweist, wobei das Rückhalteelement (28) für Feuchtigkeit zumindest teilweise durchlässig ist, wobei die Sensoranordnung (10) eine Einlassöffnung (16) für Feuchtigkeit in das Sensorgehäuse (14) aufweist und der Feuchtesensor (24) und das Rückhalteelement (28) so in dem Sensorgehäuse (14) angeordnet sind, dass der Feuchtesensor (24) über die Einlassöffnung (18) und das Rückhalteelement (28) mit Feuchtigkeit beaufschlagbar ist,
wobei die Sensoranordnung (10) mindestens eine von der Einlassöffnung (16) getrennt angeordnete Auslassöffnung (18) für Feuchtigkeit aus dem Sensorgehäuse (14) in das strömende fluide Medium aufweist, **dadurch gekennzeichnet, dass** das Sensorgehäuse (14) so ausgebildet ist, dass das fluide Medium im Bereich der Auslassöffnung (18) schneller strömt als im Bereich der Einlassöffnung (16), wobei das Sensorgehäuse (14) im Wesentlichen die Form eines Tragflächenprofils aufweist, wobei die Einlassöffnung (16) und die Auslassöffnung (18) auf einer Oberseite (36) des Tragflächenprofils angeordnet sind.

2. Sensoranordnung (10) nach dem vorhergehenden Anspruch, wobei sich in der Hauptströmungsrichtung (12) gesehen die Einlassöffnung (16) stromabwärts der Auslassöffnung (18) befindet.

3. Sensoranordnung (10) nach einem der vorhergehenden Ansprüche, wobei der Feuchtesensor (24) eine Messoberfläche (32) aufweist, wobei das Sensorgehäuse (14) so ausgebildet ist, dass innerhalb des Sensorgehäuses (14) zwischen der Einlassöffnung (16) und der Auslassöffnung (18) die Messoberfläche (32) des Feuchtesensors (24) von dem fluiden Medium parallel überströmbar ist.

4. Sensoranordnung (10) nach einem der vorhergehenden Ansprüche, wobei das Sensorgehäuse (14) einen Kanal (22) aufweist, wobei der Kanal (22) zwischen der Einlassöffnung (16) und der Auslassöffnung (18) angeordnet ist, wobei der Feuchtesensor (24) in dem Kanal (22) angeordnet ist.

5. Sensoranordnung (10) nach einem der vorhergehenden Ansprüche, wobei das Sensorgehäuse (14) eine Anströmkante (34) aufweist, die dem in der Hauptströmungsrichtung (12) strömenden fluiden Medium entgegenweist.

6. Sensoranordnung (10) nach einem der vorhergehenden Ansprüche, wobei das Sensorgehäuse (14) so ausgebildet ist, dass das fluide Medium im Bereich der Einlassöffnung (16) einen höheren statischen Druck als im Bereich der Auslassöffnung (18) aufweist.

7. Sensoranordnung (10) nach einem der vorhergehenden Ansprüche, wobei die Einlassöffnung (16) und/oder die Auslassöffnung (18) im Wesentlichen schlitzförmig ausgebildet sind.

8. Sensoranordnung (10) nach einem der vorhergehenden Ansprüche, wobei in dem Sensorgehäuse (14) ein Bypasskanal ausgebildet ist, wobei das Sensorgehäuse (14) einen Einlass in den Bypasskanal, der der Hauptströmungsrichtung (12) des fluiden Mediums entgegenweist, und mindestens einen Auslass aus dem Bypasskanal des Sensorgehäuses (14) aufweist, wobei die Sensoranordnung (10) ferner einen weiteren Sensor zur Bestimmung wenigstens eines weiteren Parameters eines durch den Bypasskanal strömenden fluiden Mediums, insbesondere einer Ansaugluftmasse einer Brennkraftmaschine, aufweist, wobei der weitere Sensor mindestens einen in dem Bypasskanal angeordneten Sensorchip zur Bestimmung des weiteren Parameters des fluiden Mediums aufweist.

## Claims

1. Sensor arrangement (10) for determining a moisture content of a fluid medium flowing in a main flow direction (12), in particular intake air of an internal combustion engine, wherein the sensor arrangement (10) has a sensor housing (14), in particular a plug-in sensor which is introduced or is able to be introduced into a flow tube, has at least one moisture sensor (24) which is arranged in the sensor housing (14) and which serves for determining the moisture content of the fluid medium, and has at least one retention element (28), wherein the retention element (28) is at least partially permeable to moisture, wherein the sensor arrangement (10) has an inlet opening (16) for moisture into the sensor housing (14), and the moisture sensor (24) and the retention element (28) are arranged in the sensor housing (14) in such a way that the moisture sensor (24) is able to be subjected to moisture via the inlet opening (18) and the retention element (28), wherein the sensor arrangement (10) has at least one outlet opening (18) for moisture from the sensor housing (14) into the flowing fluid medium, which at least one outlet opening is arranged separately from the inlet opening (16), **characterized in that** the sensor housing (14) is formed in such a way that the fluid medium flows more rapidly in the region of the outlet opening (18) than in the region of the inlet opening (16), wherein the sensor housing (14) has substantially the shape of an aerofoil-type profile, wherein the inlet opening (16) and the outlet opening (18) are arranged on a top side (36) of the aerofoil-type profile.

2. Sensor arrangement (10) according to the preceding claim, wherein the inlet opening (16) is situated downstream of the outlet opening (18) as seen in the main flow direction (12).

3. Sensor arrangement (10) according to either of the preceding claims, wherein the moisture sensor (24) has a measurement surface (32), wherein the sensor housing (14) is formed in such a way that the measurement surface (32) of the moisture sensor (24) is able to be flowed over in a parallel manner by the fluid medium within the sensor housing (14) between the inlet opening (16) and the outlet opening (18).

4. Sensor arrangement (10) according to one of the preceding claims, wherein the sensor housing (24) has a channel (14), wherein the channel (22) is arranged between the inlet opening (16) and the outlet opening (18), wherein the moisture sensor (24) is arranged in the channel (22).

5. Sensor arrangement (10) according to one of the preceding claims, wherein the sensor housing (14) has an incident-flow edge (34) which points in the direction opposite the fluid medium flowing in the main flow direction (12).

6. Sensor arrangement (10) according to one of the preceding claims, wherein the sensor housing (14) is formed in such a way that the fluid medium has a higher static pressure in the region of the inlet opening (16) than in the region of the outlet opening (18).

7. Sensor arrangement (10) according to one of the preceding claims, wherein the inlet opening (16) and/or the outlet opening (18) are/is of substantially slot-shaped form.

8. Sensor arrangement (10) according to one of the preceding claims, wherein a bypass channel is formed in the sensor housing (14), wherein the sensor housing (14) has an inlet into the bypass channel, which points in the direction opposite the main flow direction (12) of the fluid medium, and has at least one outlet from the bypass channel of the sensor housing (14), wherein the sensor arrangement (10) also has a further sensor for determining at least one further parameter of a fluid medium flowing through the bypass channel, in particular an intake-air mass of an internal combustion engine, wherein the further sensor has at least one sensor chip which is arranged in the bypass channel and which serves for determining the further parameter of the fluid medium.

## Revendications

1. Arrangement capteur (10) destiné à déterminer la teneur en humidité d'un milieu fluide qui s'écoule dans une direction d'écoulement principale (12), notamment d'un air d'aspiration d'un moteur à combustion interne, l'arrangement capteur (10) possédant un boîtier de capteur (14), notamment une sonde à enficher qui est introduite ou peut être introduite dans un tube d'écoulement, au moins un capteur d'humidité (24) disposé dans le boîtier de capteur (14) et destiné à déterminer la teneur en humidité du milieu fluide et au moins un élément de retenue (28), l'élément de retenue (28) étant au moins partiellement perméable à l'humidité, l'arrangement capteur (10) possédant une ouverture d'entrée (16) pour l'humidité dans le boîtier de capteur (14) et le capteur d'humidité (24) ainsi que l'élément de retenue (28) étant disposés dans le boîtier de capteur (14) de telle sorte que le capteur d'humidité (24) peut être exposé à de l'humidité par le biais de l'ouverture d'entrée (18) et de l'élément de retenue (28), l'arrangement capteur (10) possédant au moins une ouverture de sortie (18) disposée séparément de l'ouverture d'entrée (16) pour l'humidité issue du boîtier de capteur (14) dans le milieu fluide qui s'écoule, **caractérisé en ce que** le boîtier de capteur (14) est configuré de telle sorte que le milieu fluide s'écoule plus rapidement dans la zone de l'ouverture de sortie (18) que dans la zone de l'ouverture d'entrée (16), le boîtier de capteur (14) présentant sensiblement la forme d'un profil de surface porteuse, l'ouverture d'entrée (16) et l'ouverture de sortie (18) étant disposées sur un côté supérieur (36) du profil de surface porteuse.

2. Arrangement capteur (10) selon la revendication précédente, l'ouverture d'entrée (16), vue dans la direction d'écoulement principale (12), se trouvant en amont de l'ouverture de sortie (18).

3. Arrangement capteur (10) selon l'une des revendications précédentes, le capteur d'humidité (24) possédant une surface de mesure (32), le boîtier de capteur (14) étant configuré de telle sorte qu'à l'intérieur du boîtier de capteur (14), la surface de mesure (32) du capteur d'humidité (24) peut être submergée en parallèle par le milieu fluide entre l'ouverture d'entrée (16) et l'ouverture de sortie (18).

4. Arrangement capteur (10) selon l'une des revendications précédentes, le boîtier de capteur (14) possédant un canal (22), le canal (22) étant disposé entre l'ouverture d'entrée (16) et l'ouverture de sortie (18), le capteur d'humidité (24) étant disposé dans le canal (22).

5. Arrangement capteur (10) selon l'une des revendications précédentes, le boîtier de capteur (14) possédant un bord d'attaque (34) qui est orienté à l'opposé du milieu fluide qui s'écoule dans la direction d'écoulement principale (12).

6. Arrangement capteur (10) selon l'une des revendications précédentes, le boîtier de capteur (14) étant configuré de telle sorte que le milieu fluide possède dans la zone de l'ouverture d'entrée (16) une pression statique supérieure à celle dans la zone de l'ouverture de sortie (18).

7. Arrangement capteur (10) selon l'une des revendications précédentes, l'ouverture d'entrée (16) et/ou l'ouverture de sortie (18) étant réalisées sensiblement en forme de fente.

8. Arrangement capteur (10) selon l'une des revendications précédentes, un canal de dérivation étant formé dans le boîtier de capteur (14), le boîtier de capteur (14) possédant dans le canal de dérivation une entrée qui est orientée à l'opposé de la direction d'écoulement principale (12) du milieu fluide, et au moins une sortie hors du canal de dérivation du boîtier de capteur (14), l'arrangement capteur (10) possédant en outre un capteur supplémentaire destiné à déterminer au moins un paramètre supplémentaire d'un milieu fluide qui s'écoule à travers le canal de dérivation, notamment une masse d'air d'aspiration d'un moteur à combustion interne, le capteur supplémentaire possédant au moins une puce de capteur disposée dans le canal de dérivation en vue de déterminer le paramètre supplémentaire du milieu fluide.
